# EUROPEAN PATENT SPECIFICATION

(11) **EP 2 230 244 B1**
(45) Date of publication and mention of the grant of the patent: **07.05.2014**
(21) Application number: 09305242.1
(22) Date of filing: 17.03.2009
(51) Int. Cl.: C07F 9/50, A61K 49/10, A61K 49/12, A61K 51/06

(54) **Phosphorus polymers and their uses**
Phosphorpolymere und ihre Verwendungen
Polymères de phosphore et leurs utilisations

(43) Date of publication of application: 22.09.2010
(73) Proprietor: Ecole Polytechnique, 91128 Palaiseau Cedex (FR); Centre National de la Recherche Scientifique (CNRS), 75794 Paris Cedex 16 (FR)
(72) Inventor: Van Zutphen, Steven, 77780 Bourron Marlotte (FR); Mezailles, Nicolas, 91530, SAINT MAURICE MONTCOURONNE (FR); Le Floch, Pascal, 91400, ORSAY (FR)
(74) Representative: Le Coupanec, Pascale A.M.P.

(56) References cited:
- WO-A-96/30056
- WO-A-2009/037251
- US-A- 3 697 316
- US-A- 4 071 501
- US-A- 5 948 386
- US-A1- 2002 068 183
- HENDERSON W ET AL: "Poly(phosphine oxides) as supports for enzyme immobilisation", EUROPEAN POLYMER JOURNAL, PERGAMON PRESS LTD. OXFORD, GB, vol. 31, no. 10, 1 October 1995 (1995-10-01), pages 981-985, XP004070660, ISSN: 0014-3057, DOI: 10.1016/0014-3057(95)00054-2
- FIONA C. COCHRANE ET AL: 'Application of tris(hydroxymethyl)phosphine as a coupling agent for alcohol dehydrogenase immobilization' ENZYME AND MICROBIAL TECHNOLOGY vol. 18, no. 5, 01 April 1996, pages 373 - 378, XP055015526 DOI: 10.1016/0141-0229(95)00134-4 ISSN: 0141-0229
- TAKAFUMI SHIDO ET AL: 'EXAFS/FT-IR characterization of tetra-iridium carbonyl clusters bound to tris-(hydroxymethyl)phosphine grafted silica surface catalytically active for propene oxidation to acetone' JOURNAL OF MOLECULAR CATALYSIS A: CHEMICAL vol. 120, no. 1-3, 01 June 1997, pages 33 - 45, XP055015527 DOI: 10.1016/S1381-1169(96)00412-8 ISSN: 1381-1169
- HENDERSON W ET AL: "Immobilised phosphines incorporating the chiral biopolymers chitosan and chitin", JOURNAL OF THE CHEMICAL SOCIETY, CHEMICAL COMMUNICATIONS, CHEMICAL SOCIETY. LETCHWORTH, GB, no. 16, 1 January 1994 (1994-01-01), pages 1863-1864, XP008146923, ISSN: 0022-4936, DOI: 10.1039/C39940001863
- PETACH H H ET AL: "P(CH2OH)3 a new coupling reagent for the covalent immobilisation of enzymes", JOURNAL OF THE CHEMICAL SOCIETY, CHEMICAL COMMUNICATIONS, CHEMICAL SOCIETY. LETCHWORTH, GB, 1 January 1994 (1994-01-01), pages 2181-2182, XP008146924, ISSN: 0022-4936

## Description

The invention relates to compounds comprising at least one polymeric chain incorporating phosphorus atoms. Polymers able to coordinate metallic ions are advantageously used in a variety of applications. In particular, the compounds of the invention may be advantageously used in the coordination chemistry, in particular for capturing and complexing metal atoms.

More particularly, the compounds according to the invention may be obtained from tris(hydroxymethyl)phosphine (THP), or the likes.

Phosphines, phosphines oxide, phosphines sulphide, phosphines selenide, phosphines telluride or iminophosphoranes are already used in a great diversity of ligands able to stabilize transition metal atoms. Furthermore, those functional groups have already been incorporated in polymeric matrices, through the functionalization of polysiloxane, polystyrene or polyethylene glycol type polymers.

Henderson et al. (Eur. Polym. J., 1995, 31:981) relates to the use of tris(hydroxymethyl)phosphine (THP) reacted with 1,6-diaminohexane or Jeffamine T-403as a tool for linking an enzyme to a substrate. THP is and is used to link those amine derivatives to a support while free amine functions of the amine derivative are used to bind the enzyme. Those compounds are not used for complexing metal atoms.

Cochrane et al, (Enzyme Microb. Technol., 1996, 18:373) describes the use of a single molecule of tris(hydroxymethyl)phosphine as a coupling reagent for the immobilization of alcohol dehydrogenase onto chitosan films.

Shido et al. (Journal of Molecular Catalysis, A: Chemical 1997, 120:33) relates to the investigation of structure and catalytic activity of Ir₄(CO)₁₂ bound to tris(hydroxymethyl)phosphine grafted silica.

Also, tris(hydroxymethyl)phosphine (THP) grafted on a silica-polysiloxane support (LTS 4,157,313) were used for complexing metal atoms.

However, those compounds have, usually, a low amount of functional groups per gram of polymer.

Besides, the solubility of those compounds and their processes of preparation depend upon both of the nature of the polymer and of the nature of the functional groups. In fact, up to now, there is no simple, cheap, versatile and easily synthesized polymeric compound comprising repeated unit containing phosphorus atoms able to complex metal atoms with a high yield and which may be used in various fields as water purification, medical imaging, or chemical catalysis available.

However, it is known that hydroxymethylphosphorus compounds condense readily with amines, amides, urea, and other nitrogen-containing compounds to yield linear and branched polymers. Such compounds are used in flame retarding cotton-cellulose.

For example, in US 3,953,165 and US 4,071,501 phosphorus-containing condensation products have been proposed for treating textile articles to impart them flame retardant properties. In US 4,166,897 a process for obtaining a water-soluble phosphorus-containing condensation products containing methylene and methyl-ether type links type for treating fabrics to give them flame-retardant properties is described. US 5,948,386 discloses conjugates obtained by cross-linking one molecule with another with the reaction of hydroxymethylphosphine (HMP) with primary or secondary amine.

However, none of those polymeric compounds or materials has been proposed for complexing metal atoms.

There is a need for polymers able to bind high amount of metal atoms, in particular transition metal atoms, and to be stable over a wide range of pH.

There is a need for versatile polymers, water-soluble or insoluble, or soluble in organic solvents, preferably obtained through a unique, simple, fast and cheap process using non toxic solvents, and in particular using water.

There is a need for a versatile polymer able to complex metal atoms, in particular transition metal atoms, which may be used for purifying water, in medical imaging or as catalytic agent.

The current invention has for purpose to meet theses needs.

According to one aspect, the present invention concerns with the use of a compound comprising at least one polymeric chain, said polymeric chain incorporating phosphorus atoms and consisting, in all or in part, of identical or different repeated units, each of said units being represented by the following formula (I): 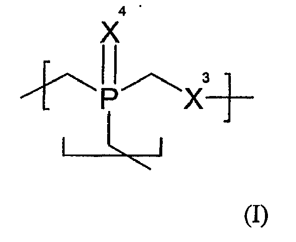 wherein:
- X³ may represent:
   - O-[Si(R¹R²)O]- with R¹ and R² being, independently of each other, a C₁-C₃₀ alkyl or alkoxy group, a C₅-C₃₀ aryl group, or
   - a mono or polyorganosilicate derived radical, or
   - N(R³)- with R³ being -H, a C₁-C₃₀ alkyl group or a C₅-C₃₀ aryl group, optionally substituted with -OH or -NH₂, or at least one unit of general formula (I), and
   - =X⁴ may represent an electron pair, =O, =S, =NR⁴ with R⁴ representing a C₁-C₃₀ alkyl group or a C₅-C₃₀ aryl group, for complexing metal atoms.

Within the invention, the terms "polymer" and "polymeric chain" are used interchangeably.

According to one embodiment, the invention relates to a compound comprising at least one polymeric chain as disclosed here above liable to be obtained according to a method comprising at least the step of contacting a first reactant P(CH₂Z)₃, wherein each Z represents, independently of each other, -OH or -CH₂OH, with a second reactant comprising at least two reactive sites chosen from "NH" and "SiOH", wherein each reactive site is liable to react with one first reactant, wherein said first reactant and said reactive sites are used in an amount equal or greater than 1 eguivalent, and more particularly ranging from 1 to 1.4 equivalents, and more preferably ranging from 1.1 to 1.3 equivalents.

According to a specific embodiment, a compound of the invention comprises a linear polymeric chain.

According to another specific embodiment, a polymer of the invention may have a tri-dimensional structure comprising several polymeric chains according to the invention.

More particularly, a polymer of the invention may be cross-linked.

According to an embodiment, the invention relates more particularly to the use of at least one a compound of the invention comprising at least one polymeric chain as above-defined for complexing metal atoms from a solution, such as water, in particular in the form of dissolved metal atoms.

An embodiment of the invention relates to a water-purifying device comprising at least one compound of the invention.

According to another embodiment, the invention relates to the use of at least one a compound of the invention comprising at least one polymeric chain as above-defined for detecting metal atoms.

In another particular embodiment, the invention relates to a water insoluble compound comprising at least one polymeric chain, said polymeric chain incorporating phosphorus atoms and being represented by the following formula (II): 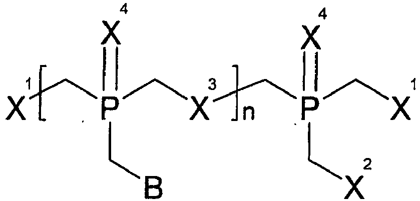 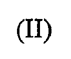 wherein n, B, X¹, X², X³ and =X⁴ are as defined hereafter.

The polymers of the invention are particularly interesting with respect to their ability to bind metal atoms, in particular transition metal atoms, through coordination bonds resulting in high specificity and selectivity.

The complexed metal atoms of the invention may be in the form of ions or metal oxide, or neutral metal species.

As stated here-after a polymer of the invention may comprise as functional group having electron pairs a great diversity of groups that may be chosen from groups of trialkylphosphine type, trialkylphosphine oxide type, trialkylphosphine sulphide type or iminophosphorane type.

Therefore the choice of a convenient polymer according to the metal with which a bond may be formed may be easily made in order to bind therein one metal atom rather than another.

By way of example, copper (I) or (II) ions will preferably be complexed with phosphine type polymer, whereas zirconium (IV) ions will preferably be complexed with phosphine oxide type polymer.

Thus, according to another aspect the invention relates to a metal-based coordination complex comprising at least one metal atom complexed via coordination bonds with at least one compound of the invention.

The current invention relates, according to another embodiment, to a stabilized nanoparticle of metal atoms, in particular of aggregated metal atoms, coated at least in part with at least one compound of the invention.

Another embodiment of the invention relates to a compound for medical imaging comprising at least one metal-based coordination complex of the invention or at least one stabilized nanoparticle of metal atoms of the invention, in particular of aggregated metal atoms.

Another embodiment of the invention relates to a catalytic agent comprising at least one metal-based coordination complex of the invention or comprising at least one stabilized nanoparticle of metal atoms of the invention, in particular of aggregated metal atoms.

### POLYMER

A compound of the invention comprises at least one polymeric chain incorporating phosphorus atoms.

A compound according to the invention comprises at least one polymeric chain which incorporates phosphorus atoms and which may consist, in all or in part, of identical or different repeated units, each of said units may be represented by the following formula (I): 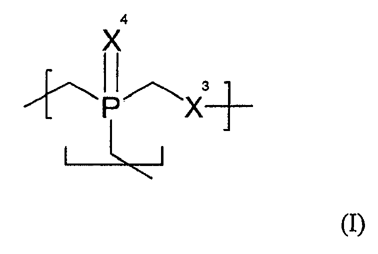 wherein:
- X³ may represent:
   - O-[Si(R¹R²)O]- with R¹ and R² being, independently of each other, a C₁-C₃₀ alkyl or alkoxy group, a C₄-C₃₀ aryl group, or
   - a mono- or polyorganosilicate derived radical, or
   - N(R³)- with R³ being -H, a C₁-C₃₀ alkyl group or a C₅-C₃₀ aryl group, optionally substituted with -OH or -NH₂, or at least one unit of general formula (I), and
   - =X⁴ may represent an electron pair, =O, =S, =NR⁴ with R⁴ representing a C₁-C₃₀ alkyl group or a C₅-C₃₀ aryl group, for complexing metal atoms.

Within the meaning of the invention, "alkyl" intends to mean linear, branched, saturated alkyl radicals.

Within the meaning of the invention, "alkoxy" intends to mean a radical -OR wherein R represents a linear, branched, saturated alkyl radical.

Within the meaning of the invention, "aryl" intends to mean monocyclic or polycyclic, condensed or not, radical. According to an embodiment, an aryl group of the invention may be a 5-30 members heteroaryl group, i.e an aryl group comprising one or more heteroatom such as O, S or N interrupting its hydrocarbon chain.

According to one embodiment, R¹, R², R³ and R⁴ may, independently of each other, represent a linear, branched, saturated C₁-C₃₀ alkyl group, in particular a C₂-C₂₀, more particularly a C₃-C₁₈, more particularly a C₄-C₁₆ and more particularly a C₈-C₁₂ alkyl group, or a C₅-C₃₀ aryl group, and in particular a C₁₀-C₂₀, and more particularly a C₁₄-C₁₈ aryl group, or 5-30 members heteroaryl group, and in particular 10-20 members, and more particularly 14-18 members heteroaryl group.

According to another embodiment, R¹ and R² may, independently of each other, represent a linear, branched, saturated C₁-C₃₀ alkoxy group, in particular a C₂-C₂₀, more particularly a C₃-C₁₈, more particularly a C₄-C₁₆ and more particularly a C₈-C₁₂ alkoxy group.

According to a preferred embodiment, X³ may represent -N(R³)- where R³ is different from H.

According to a preferred embodiment, R³ may represent an alkyl or an aryl or an heteroaryl group as above-defined, optionally substituted with at least one radical chosen from OH or NH₂, and in particular with OH.

In particular, R³ may represent a C₁-C₆, more particularly a C₂-C₅, and more particularly, a C₃-C₄ alkyl group, optionally substituted with at least one radical chosen from OH or NH₂, and in particular with OH.

According to a preferred embodiment, R³ may represent at least one unit of general formula (I). In such embodiment, the free extremities of a unit figured by formula (I) which are not engaged in the polymeric chain may be substituted with X¹ or X² as defined below.

Preferably, R³ may figure a radical comprising repeated units figured by formula (I). The number of repeated units may range from 1 to 10⁶, preferably from 10 to 10⁵, and more preferably from 100 to 10⁴. The repeated units of formula (I) figuring R³ may represent a polymeric chain.

R³ may figure a linear, branched or cross-linked polymeric chain. Owing to the structure of the repeated unit of formula (I), the branches of the polymeric chain figured by R³ may be represented by at least one unit of formula (I), and preferably by repeated units of formula (I). The number of repeated units may be as above-defined.

According to one embodiment each X³ moiety of each repeated unit may be different or identical one relative to each other.

According to a preferred embodiment, =X⁴ may represent =O, =S, or an electron pair, and more particularly may be an electron pair.

According to one embodiment each X⁴ moiety of each repeated unit may be different or identical one relative to each other.

Preferably, a polymeric chain of the invention may comprise at least two, preferably three and more preferably four different repeated X⁴ moieties.

Advantageously, a polymeric chain of the invention may be represented by the following formula (II): 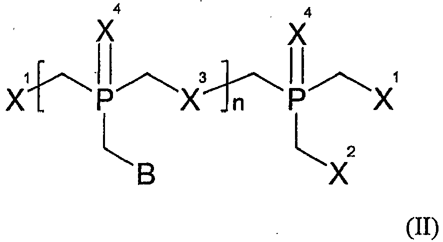 wherein:
- n may range from 1 to 10⁶, in particular from 10 to 10⁵, and more particularly from 100 to 10⁴,
- X³ and =X⁴ may be as above-defined, with each X³ being identical to each other when n is greater than 1, and when X³ represents -NR³- with R³ being at least one unit of general formula (I) then the free extremities of said at least one unit which are not engaged in said polymeric chain are substituted with X¹ or X² as defined below,
- X¹ and X² may represent, independently of each other, -OH, NH₂, -NHR⁵ or -OSiR⁶R⁷(OH), with R⁵, R⁶ and R⁷ being, independently of each other, a C₁- C₃₀ alkyl group or a C₅-C₃₀ aryl group, or R⁶ being as previously defined and R⁷ figuring -OCH₂-B, with B representing a radical of formula (III): 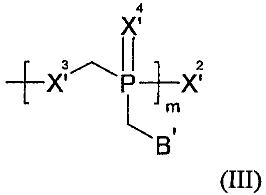 wherein:
   - m may range from 0 to 10⁶, in particular from 10 to 10⁵, and more particularly from 100 to 10⁴, and
   - B' may represent B, with each B being identical to each other when m is different from 0,
   - X'² may represent X² as above-defined, X'³ may represent X³ as above-defined and =X'⁴ may represent =X⁴ as above-defined, with each X'³ being identical relative to each other when m is different from 0.

According to one embodiment, R⁵, R⁶ and R⁷ may, independently of each other, represent a linear, branched, saturated C₁-C₃₀ alkyl group, in particular a C₂-C₂₀, more particularly a C₃-C₁₈, more particularly a C₄-C₁₆ and more particularly a C₈-C₁₂ alkyl group, or a C₅-C₃₀ aryl group, and in particular a C₁₀-C₂₀, and more particularly a C₁₄-C₁₈ aryl group.

According to another embodiment R⁶ may be as above-defined and R⁷ may figure -OCH₂-B, with B being as above defined.

According to one embodiment, X¹ and X² may represent, independently of each other, -OH, -NHR⁵, or -OSiR⁶R⁷(OH), with R⁵, R⁶ and R⁷ being as above defined.

In a particular embodiment, the invention has for object a compound comprising at least one polymeric chain of formula (II) wherein n, B, X¹, X² and X³ are as previously defined and =X⁴ is =O, =S or an electron pair, and with each X³ being identical relative to each other when n is greater than 1.

More preferably, a compound according to the invention may comprise at least one polymeric chain represented by the following formula (IV): 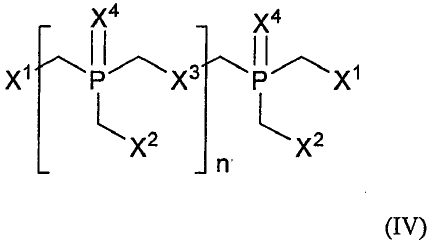 wherein n, X¹, X², X³ and =X⁴ are as above-defined.

According to another embodiment, a compound according to the invention may comprise at least one polymeric chain represented by the following formula (V): 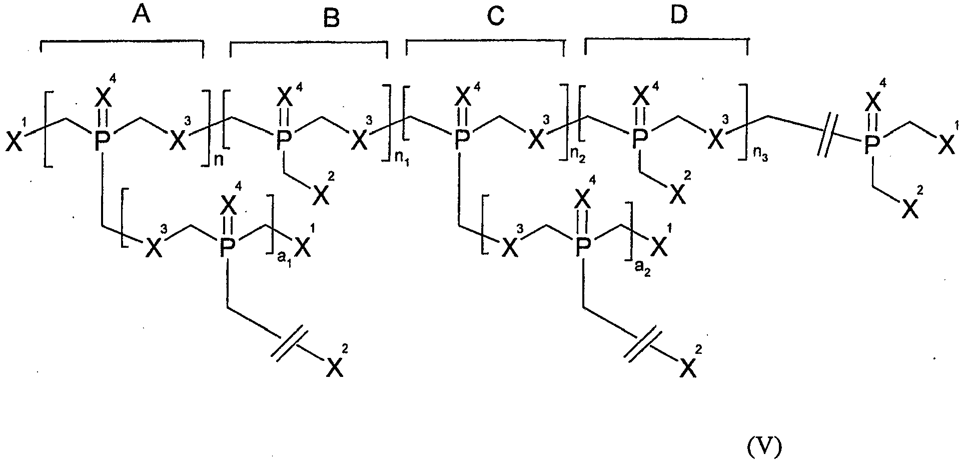 wherein:
- n, X¹, X², X³ and =X⁴ may be as above-defined,
- n₁, n₂ and n₃ may range, independently of each other, from 0 to 10⁶, in particular from 10 to 10⁵, and more particularly from 100 to 10⁴, and
- a₁ and a₂ may range from 1 to 10⁶, in particular from 10 to 10⁵, and more particularly from 100 to 10⁴,
- -//- figures an optional continuation of said polymeric chain, and
- A, B, C and D may be randomly or sequentially distributed.

According to another embodiment, a compound according to the invention may be as above-defined with X¹ and X² being identical.

According to one embodiment, a polymer of the invention may preferably comprise at least one polymeric chain comprising at least one group chosen from groups of trialkylphosphine type, trialkylphosphine oxide type, trialkylphosphine sulphide type or iminophosphorane type, and more preferably chosen from trialkylphosphine type or trialkylphosphine oxide type. 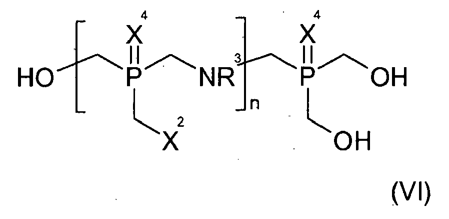 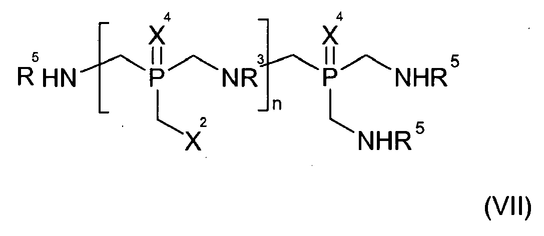 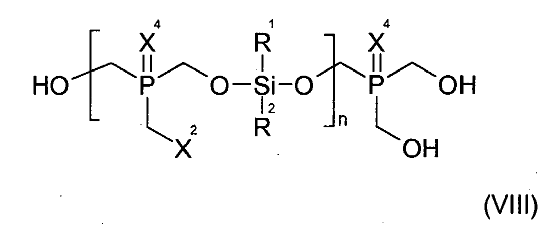 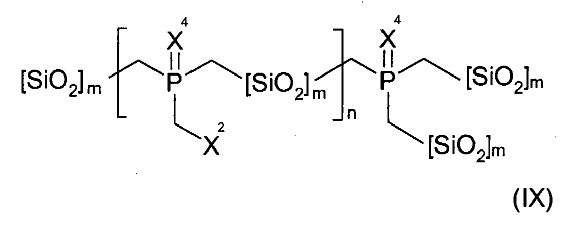 wherein, B, =X⁴, R¹, R², R³, R⁵ and n are as above-defined.

According to an embodiment, a compound of the invention may comprise at least one polymeric chain as previously defined where X1 and X2 represent -NH₂, X3 represents -NH- or -NR³- with R³ being of formula (I), and =X4 represents an electron pair; or where X1 and X2 represent -NH₂, X3 represents -NH- or -NR³- with R³ being of formula (I), and =X4 represents =O; or where X1 and X2 represent -NHC₈H₁₇, X3 represents -N(C₈H₁₇)-, and =X4 represents an electron pair; or where X1 and X2 represent -NHC₁₂H₂₅, X3 represents -N(C₁₂H₂₅)-, and =X4 represents an electron pair.

According to another embodiment, a compound according to the invention may be insoluble in water. The solubility of a compound of the invention may be determined by any known techniques in the art.

According to another embodiment, a compound of the invention may be grafted onto a support of mineral or organic material.

As example of mineral material suitable for the invention, one may mention material chosen from the group consisting of a metal oxide such as TiO₂ or SiO₂.

The grafting of a compound of the invention onto a mineral material may be carried by any known methods in the art.

For example, after an activation of the mineral surface of the support, by, for example, oxygen plasma treatment or pyrolysis, the monomer THP only, or THP in the presence of a second reactant such as an amine, for example NH₄OH, is presented to the mineral material. Reaction of the monomer with surface MOH (where M is the metal for example Si or Ti) and subsequent reaction of the second and third CH₂Z groups on the monomer with other MOH groups or with the other second reactant will lead to the formation of polymers grafted to the mineral material.

As example of organic material suitable for the invention, one may mention organic material chosen from the group consisting of cellulose fibers, paper, plastic, mineral materials functionalized with APS or the like, amine containing reagent, amine containing organic polymer, or biological amine containing polymers such as polylysine.

The grafting of a compound of the invention onto an organic material may be also carried by any known methods in the art.

For example, the monomer THP only, or THP in the presence of a second reactant such as an amine, for example NH₄OH, is presented to the organic material. Reaction of the monomer with surface amines and subsequent reaction of the second and third CH₂Z groups on the monomer with other surface amines or with the other second reactant will lead to the formation of polymers grafted to the organic material.

In one embodiment, a support onto which a compound of the invention may be grafted may be in the form of a particle or a bead, or may be a surface or the surface of devices such as a wall of a channel or a microchannel, or a micro or macroporous material.

### METAL-BASED COORDINATION COMPLEX

An object of the present invention is a metal-based coordination complex, comprising at least one metal atom and at least one compound of the invention, the said metal atoms being bound to the polymer of the invention via coordination bonds.

Typically, a metal-based coordination complex of the invention comprises a polymer comprising groups which may in particular be based on phosphorus comprising groups capable of developing Lewis acid-Lewis base bonds with metal atoms and thus opens the way to numerous applications.

In a complex of the invention, a metal atom may be in the form of an oxide or a salt or a neutral solvate species.

A metal-based coordination complex of the invention may be obtained by reacting a compound of the invention with a salt or an oxide of the metal atom or another solvate species to be complexed in order to obtain the expected complex or one of its salts with an appropriate counter-ion.

Metal atoms that may be complexed with a compound of the invention may be, for example, an oxide or a salt or a neutral solvate of a paramagnetic metal of atomic number 21-29, 42- 44 or 58-70 (for example, scandium, titanium, vanadium, chromium, manganese, iron, cobalt, nickel, copper, molybdenum, ruthenium, cerium, praseodymium, neodymium, promethium, samarium, europium, gadolinium, terbium, dysprosium, holmium, erbium, thulium, and ytterbium; the elements Gd(III), Mn(II), europium and dysprosium are particularly preferred), or a radionuclide chosen from ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh, ¹⁸⁸Re, ⁶⁰Cu, ⁶²Cu, ⁶⁴Cu, ⁶⁷Cu, ⁹⁰Y, ¹⁵⁹Gd, ¹⁴⁹Pr and ¹⁶⁶Ho, or an ion of a heavy metal of atomic number 21-31, 39-49, 50, 56-80, 82, 83 or 90.

In particular, metal atoms convening for the invention may be chosen from Ti, V, Cr, Mn, Fe, Co, Ni, Cu, Zn, Y, Zr, Hf, Nb, Mo, Cd, Ta, W et Re; and rare earth metal such as La, Ce, Pr, Nd, Sm, Eu, Tb, Th, Dy and U, or noble or platinum group metals chosen from Pb, Pd, Pt, Au, Ru, Os, Ag, Rh, or Ir.

Advantageously, a complex according to the present invention may comprise an metal atom or an ion of a paramagnetic metal chosen from Gd³⁺, Mn²⁺ and Fe³⁺.

According to one embodiment, a metal-based coordination complex may comprise a compound of the invention comprising at least one polymeric chain comprising at least trialkylphosphine groups and metal atoms rich in electrons, such as for example platinum.

According to another embodiment, a metal-based coordination complex may comprise a compound of the invention comprising at least one polymeric chain comprising at least phosphine oxide groups and metal atoms poor in electrons, such as for example zirconium.

According to another embodiment, the metal atoms may be in the form of nanoparticles, or part of said nanoparticles, stabilized by at least one compound of the invention. Within the meaning of the invention, the term "nanoparticle" is intended to mean assemblies of atoms of nanometric size. In a particular embodiment, a nanoparticle of the invention may be made of aggregated metal atoms.

Nanoparticles may be pure metal nanoparticles, such as group XI metal centers (Au, Ag, or Cu) or a binary mixture of metal atom and another element such as luminescent semi-conductors (group II-VI or III-V, such as InP for example).

Advantageously, metal nanoparticles of the invention may comprise Au nanoparticles, often used in biodiagnostics as described by Rosi & Mirkin in Chem. Rev., 2005,105:1547.

### APPLICATIONS

As stated previously, the present invention relates to the use of a compound of the invention for very different applications which relate to the field of coordination chemistry.

These applications are highly varied and relate in particular to the field of catalysis, the field of the extraction or recycling of metals, and in particular metal atoms, the field of detecting and analysing metal atoms, the field of the stabilization of nanoparticles, the field of catalysis as well as the field of medical imaging.

Thus, the present invention relates to the use of at least one compound of the invention, in particular comprising at least one polymer obtained from tris(hydroxymethyl)phosphine (THP), for their intrinsic properties of forming bonds with metal atoms, in particular with transition metal atoms.

### Extraction, recycling or detection of metals

The coordination chemistry of metal atoms has numerous applications, such as the extraction and recycling of metal atoms, and in particular transition metal atoms.

In these fields, the aim is to form scavengers for metals, generally in solution. For this, the aim is to find entities capable of easily forming bonds with metals atoms and then to recover said metals atoms. According to the invention, when a polymer-metal complex has been formed by Lewis base-Lewis acid interactions, it is possible to recover the metal atoms by filtration or extraction.

Therefore, the current invention has for object the use of a compound as above-defined for complexing metal atoms. In particular, a use of the invention may be carried out on metal atoms present in a solution, such as an aqueous solution.

An aspect of the invention relates to a use of a compound of the invention for purifying a solution, and in particular an aqueous-solution such as wasted-water.

In a preferred embodiment, a use of the invention may be carried out for complexing metal atoms from water.

Accordingly, another subject-matter of the invention is a method for purifying a solution presumed comprising metal atoms comprising at least the steps consisting of:
- introducing a compound according to the invention into a solution presumed comprising metal atoms in conditions suitable for obtaining at least one complex of metal-polymer type,
- removing said complex from the solution, for example by centrifuging or filtering, and
- recovering said solution substantially devoid of metal atoms.

Within the meaning of the invention, "substantially" intends to mean that the solution treated according to the invention does not comprise any longer detectable amount of dissolved metal atoms, as it may be detected with usual methods known in the field.

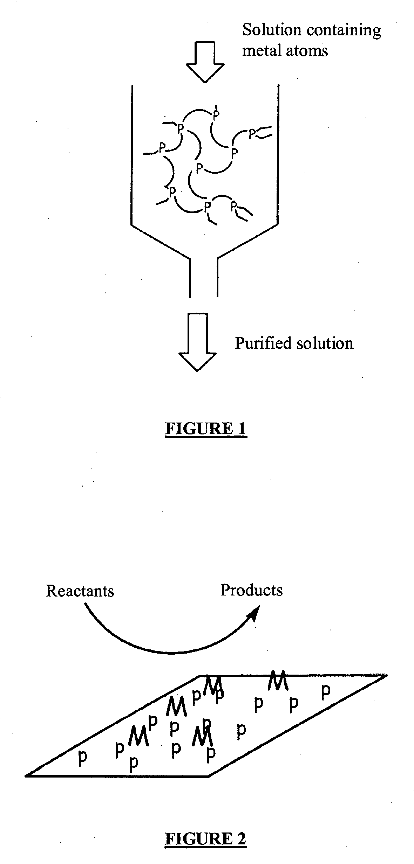

Figure 1 illustrates, as such, an example of a reaction scheme used during the purifying method of the invention, using a complex of the invention and a solution loaded with dissolved metal particles.

A first solution comprising solubilised metal atoms under the form of metal oxide or metal salt is introduced into a medium comprising a compound of the invention resulting in the formation of a second solution. This second solution is filtered, making it possible to isolate, on the filter, the compound-metal complex formed *in situ* in the second solution and allowing the passage of a solution purified from metal atoms.

Such a purification method is particularly direct and no longer requires the use of resins generally employed according to the known art.

In numerous applications of the waste treatment type, for example radioactive waste treatment type, there is a need to extract metal entities such metal atoms. For this, a compound of the invention may be contacted with a solution comprising such entities. The metal-based complex obtained is then recovered, for example by filtration or centrifugation, and then heated at a temperature at which the organic species decompose, in order to collect the metal.

Such a process is particularly advantageous in so far as it is very direct and in so far as it makes it possible to treat large amounts of entities to be recycled.

An aspect of the invention relates to a use of a compound of the invention for recycling metal atoms.

Therefore, a further subject-matter of the invention is a method for recycling metal atoms comprising at least the steps consisting of:
- providing a solution comprising metal atoms,
- introducing in said solution a compound according to the invention in conditions suitable for obtaining at least one complex of metal-polymer type,
- removing said complex from the solution, for example by centrifuging or filtering,
- recovering said metal atoms from said complex, for example by dissolving said complex.

According to one embodiment, the recovering step may be carried by heating the solution comprising said complex to a temperature greater than the decomposition temperature of the compound of the invention so as to obtain a mixture of decomposed products.

The recovering of said metal atoms may be further carried by filtration of said mixture of heated and decomposed products.

It is then easy and inexpensive to recover the metal atoms by dissolution or by incinerating the combination at a temperature at which the organic species decompose.

An aspect of the invention relates to a use of a compound of the invention for detecting metal atoms.

Therefore, another subject-matter of the invention is a method for detecting a solution presumed comprising metal atoms comprising at least the steps of:

An aspect of the invention relates to a use of a compound of the invention for detecting metal atoms.

Therefore, another subject-matter of the invention is a method for detecting a solution presumed comprising metal atoms comprising at least the steps of:
- introducing a compound according to the invention into a solution presumed comprising metal atoms in conditions suitable for obtaining at least one complex of metal-polymer type,
- recovering said complex from the solution, for example by centrifuging or filtering, and
- detecting the metal atoms in said complex.

The detecting step may be carried out *in situ* in said complex or after a step of extracting the metal atoms from said complex. The extracting step may be carried out for example by heating said complex to a temperature greater than the decomposition temperature of the compound of the invention as indicated above.

The step of detecting may be carried out by any techniques known in the art, for example by spectroscopy.

Advantageously, after the step of introducing a compound of the invention in a solution and before the step of removing the said complex, a method of the invention may comprise a step of altering the pH of said solution, either by lowering or by increasing said pH in order to improve the precipitation of said complex.

Preferably the pH may be lowered. The modification of the pH of a solution may be carried out by any techniques known in the art, for example by introducing a base or an acid in said solution.

According to an embodiment, a compound of the invention that may be used in the above-described methods may preferably comprise a polymer comprising repeated units of trialkylphosphine type, of trialkylphosphine oxide type, of trialkylphosphine sulphide type, or of iminophosphorane type.

According to an embodiment, the formation of a complex of metal-polymer type may be carried out either by introducing a first solution presumed comprising metal atoms into a second solution comprising a compound of the invention or by introducing a

According to a preferred embodiment, a compound of the invention that may be used in the methods of the invention may be grafted onto a support as above indicated.

According to another preferred embodiment, a compound of the invention may be grafted or bound to a surface for example of a device, a bead or a particle. The support brings advantages in terms of handling of the compound, pressure drop properties of a filtering device, and easy of separation of the complex from the solution.

According to one embodiment, the present invention relates to a filtering-device comprising at least one compound of the invention. Such filtering-device may be preferably used for water purification.

For example, a compound of the invention may be placed on a filter with a pore size smaller than the particle size of the compound of the invention. Water presumed to contain metal atoms can than be flowed through the device allowing the formation of coordination bonds between the compound of the invention and the metal atoms. Water presumed devoid of selected metal atoms is then recovered at the end of the filter.

### Catalytic agent

Another object of the invention is the use of a coordination complex according to the invention as catalytic agent, or catalyst, for chemical synthesis.

According to one of its object, the invention relates to a catalytic agent comprising at least one metal-based complex of the invention or comprising at least one stabilized nanoparticle of metal atoms of the invention, in particular one nanoparticle of aggregated metal atoms.

In the field of catalysis, the advantage of the polymer-metal centres combination is that of lying at the boundary between homogeneous catalysis and heterogeneous catalysis. This is because the polymer-metal centres combination can be chosen for its solubility in a given solvent, which makes it possible to carry out a reaction in a homogeneous phase. There thus exists good contact between the catalyst and the solution, the surface of the polymer conferring a defined electron and steric environment to the metal which makes possible high activity and selectivity.

Therefore, compounds of the present invention present the advantage of being soluble in various solvents, including water or an organic solvent, such as toluene, while being easy to isolate so as to be able to be removed at the end of a chemical reaction.

An advantage the invention is that a soluble compound of the invention may be more easily precipitated or else extracted with a selected solvent from a reaction medium than a conventional, homogeneous catalytic agent.

According to another embodiment, the invention relates to a chemical synthesis method comprising at least a step of reacting at least two reactants in presence of at least one catalytic agent of the invention.

In preferred embodiments, metal atoms which may be advantageously used in a catalytic agent of the invention may be chosen from Pd, Pt, Rh, Ru, Co, Fe, Ni or another suitable transition metal species or combination thereof.

As illustrated in Figure 2, a compound of the invention is introduced into a solution comprising reactants in the presence of metal atoms M as catalytic precursor. It can typically be a metal salt in solution. The polymer and the catalytic precursor form a catalytic complex.

The reaction between reactants takes place in the presence of the catalytic complex. It is possible to combine the advantages of a catalytic agent which is soluble in the reactive medium and the possibility of isolating said catalytic agent at the end of the reaction by filtration or by extraction with solvents.

### Medical imaging

Another object of the invention is the use of a metal-based coordination complex according to the invention or of a stabilized nanoparticle of metal atoms of the invention, in particular of aggregated metal atoms, as a compound for medical imaging, and in particular as contrast agent.

Today, one of the major hurdles in medical imaging relates to the solubility of nanoparticles, as "marker", in biological media. This is mainly because nanoparticles are synthesized in organic solvents and have low-water solubility properties whereas it is an absolute necessity to obtain an adequate level of dissolution of those nanoparticles in water, and in particular in biological fluids such as blood. The already considered techniques of attaching water-soluble ligands to those nanoparticles, which are complex and expensive, have not yet provided satisfying results. absolute necessity to obtain an adequate level of dissolution of those nanoparticles in water, and in particular in biological fluids such as blood. The already considered techniques of attaching water-soluble ligands to those nanoparticles, which are complex and expensive, have not yet provided satisfying results.

The coordination chemistry of polymers based on phosphines also has particularly advantageous applications in the field of medical imaging using nanoparticles of metal atoms, in particular of aggregated metal atoms,

Accordingly, a further subject-matter of the invention is a compound for medical imaging, in particular a contrast agent, comprising a metal-based coordination complex according to the invention or a stabilized nanoparticle of metal atoms of the invention, in particular of aggregated metal atoms.

Advantageously the compound of the invention for medical imaging is water soluble.

In preferred embodiments, metal atoms which may be advantageously used in medical imaging may be chosen from nanoparticles of metals of group XI (Ag, Au or Cu, for example) or nanoparticles of semi-conductors II-VI or III-V.

According to one embodiment, a compound of the invention may be modified with functional reacting groups capable of creating bonds with a support of biological type, in particular with biomolecule.

As functional reacting groups one may mention the radical -CH₂OH which may be a substituent of phosphorus atoms incorporated in a polymer of the invention.

Compounds of the invention comprising groups of trialkylphosphine type, trialkylphosphine oxide type , trialkylphosphine sulphide type exhibit the great advantage of being soluble, depending on the substituents of the amine, in solvents and in particular in water.

According to one embodiment, the invention relates to a method of preparing water-soluble stabilised nanoparticles of metal atoms, in particular of aggregated metal atoms, for medical imaging comprising at least the steps of:
- providing a solution comprising dissolved metal atoms as metal precursors,
- contacting said solution with least one compound of the invention in conditions suitable for the aggregating of said metal precursors into nanoparticles and the coating of said nanoparticles with said polymer.

It is of routine tasks for a skilled artisan to select suitable conditions for

Alternatively, a method of the invention may comprise at least the steps of:
- providing a solution comprising solubilised preformed nanoparticles,
- contacting said solution with at least one compound of the invention comprising at least one polymeric chain of the invention, in conditions suitable for the coating of said nanoparticles with said compound.

The nanoparticles thus obtained may comprise about ten, twenty or more preferably thirty to about four or five thousand metal atoms, in particular as aggregated metal atoms.

One advantage of the method of the invention is the formation *in situ* of nanoparticles coated with compounds of the invention.

According to another embodiment, a method of the invention may comprise at least a step of preparing nanoparticles of metal atoms, in particular of aggregated metal atoms, a step of coating said nanaoparticles with "monodentate" ligands to obtain stabilized nanoparticles, a step of contacting said stabilized nanoparticles with a compound of the invention, preferably in solution, to obtain the replacement of the "monodentate" ligands by "polydentate" ligands composed of said compound.

Said method allows advantageously providing simultaneously the nanoparticles with stabilization and dissolution functions.

### PREPARATION OF COMPOUNDS

The current invention proposes also to a method for synthesizing a compound of the invention incorporating phosphines groups.

As starting material providing phosphine groups one may use the tetra(hydroxymethyl)phosphonium chloride, [P(CH₂OH)₄]Cl (THPC), which may be transformed into a first reactant, tris(hydroxymethyl)phosphine, P(CH₂OH)₃, called THP, using a variety of bases or reducing agents, such as sodium hydroxide, ammonia or an organic base such as triethylamine. Advantageously, a tris(hydroxymethyl)phosphine (THP) group may be obtained by neutralizing tetra(hydroxymethyl)phosphonium chloride with sodium hydroxyde (NaOH).

The different compounds considered according to the invention may be obtained by reaction, in particular by co-polymerization, of the first reactant with a second reactant intended to provide the group X3 considered in the formulae of the polymer of the with sodium hydroxyde (NaOH).

The different compounds considered according to the invention may be obtained by reaction, in particular by co-polymerization, of the first reactant with a second reactant intended to provide the group X3 considered in the formulae of the polymer of the invention.

A second reactant suitable for the invention may comprise at least two reactive sites, each liable to react with one first reactant in conditions suitable for reacting, in particular co-polymerizing, the first reactant with the second reactant. A reactive site suitable for the invention may be in particular chosen from "NH" or "SiOH".

According to an embodiment, the two requested reactive sites may be borne in one nucleophilic moiety or may be borne in two distinct nucleophilic moieties. A nucleophilic moiety may comprise at least one reactive site, preferably two reactive sites, more preferably three and more preferably four reactive sites.

Within the meaning of the invention, a reactive site is intended to figure all or part of a nucleophilic moiety able to enter in a reaction with a first reactant.

A nucleophilic moiety may be chosen from NH, NH₂, NH₃, NH₄⁺, SiOH or Si(OH)₂.

A nucleophilic moiety such as NH comprises only one reactive site "NH", and in such case the nucleophilic moiety figures the reactive site in its whole. Furthermore in such a case the second reactive site requested according to the invention will have to be provided by another nucleophilic moiety present in said second reactant so as to allow the reaction, and in particular the co-polymerization, of a first reactant with a second reactant.

By contrast, the nucleophilic moiety NH₂ comprises directly two reactive sites "NH", NH₃ comprises three reactive sites "NH", NH₄⁺ comprises four reactive sites "NH", SiOH comprises one reactive site "SiOH" and SiOH₂ comprises two reactive sites "SiOH".

A second reactant suitable for the invention may be chosen from NH₂R with R representing a C₁-C₃₀ alkyl, optionally substituted with -OH or -NH₂, or a C₅-C₃₀ aryl group; silica derivatives such as SiYₙZₚ, with n+p=4, and Y figuring a radical chosen from OH, and Z figuring a radical chosen from a C₁-C₅ alkyl group or a C₅-C₃₀ aryl group. In particular, a second reactant may be chosen from NH₂-R- NH₂, NH(R)-R-NH₂, NH(R)-R-NH(R) with R representing a C₁-C₃₀ alkyl or alkylene group, optionally substituted with -OH or -NH₂, or a C₅-C₃₀ aryl or arylene group. Also, a second reactant may be NH₃, NH₄OH or NH₄Cl.

According to a preferred embodiment, a first reactant, such as P(CH₂Z)₃, and a second reactant comprising the said reactive sites are used in amounts such that the first reactant and the reactives sites are in an amount equal or greater than 1 equivalent, in particular ranging from 1 to 1.4 equivalents, and more preferably ranging from 1.1 to 1.3 equivalents.

The structure of a compound of the invention i.e. linear or tri-dimensional, cross-linked or not, as represented in formula (V) may be adjusted through the weight ratio between the first and second reactants.

A reaction according to the invention may be carried out in water when oxygen is present, without oxidation of THP. Only few intermediates are present.

More particularly, the preparation of a compound of the invention incorporating phosphines groups and amino groups, may comprise at least a step of reacting, as first reactant, a tris(hydroxymethyl)phosphine (THP) with, as second reactant, an ammonium group, a primary or a secondary amine.

The OH groups of THP react with amines according to a Mannich-type condensation reaction (Tetrahedron, 46, 1791, 1990).

When using secondary amine, NHR₂, three clearly defined products (R₂NCH₂)P(CH₂OH)₂, (R₂NCH₂)₂P(CH₂OH) and (R₂NCH₂)₃P may be formed depending on the added amine proportion.

Using primary amine, NH₂R, in the same reaction, the defined product (RNHCH₂)₃P may be isolated if the amine is added to great excess.

When a smaller amount of amine is used, polymer species form quickly.

The optimum ratio of THP/amine is 1 or more.

When using primary amine, solubility may be controlled by the nature R group of the NHR₂ group.

As example, with a phenyl-type R group, a compound of the invention may be soluble in acetone.

With a long-chain alkyl R group, a compound of the invention may be soluble in solvents, such as toluene or chloroform or THF (tetrahydrofuran).

With a polar R group, as for example -R'CH₂COOH, a compound of the invention may be soluble in water.

According to one embodiment, a compound of the invention incorporating phosphorus atoms, for example in the form of phosphines groups, may be obtained by reacting a primary amine with tris(hydroxymethyl)phosphine (THP) groups.

The reaction of one equivalent of RNH₂ yields a polymer having -CH₂OH end extremities.

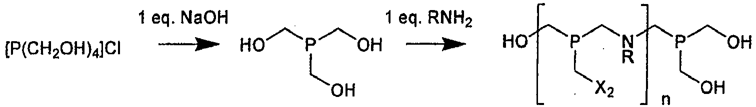

The reaction with amount greater than one equivalent of RNH₂ yields a polymer having -CH₂NHR end extremities.

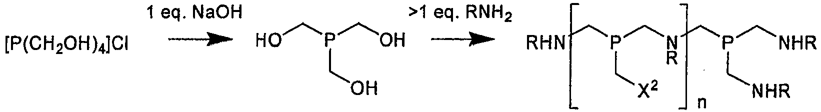

Regarding more particularly the preparation of a compound of the invention incorporating phosphines groups and silica groups, the method of the invention may comprise at least a step of reacting, as first reactant, a tris(hydroxymethyl)phosphine (THP) with, as second reactant, silica derivatives such as SiYₙZₚ, with n+p=4, and Y figuring a radical chosen from OH, and Z figuring a radical chosen from a C₁-C₅ alkyl group or a C₅-C₃₀ aryl group.

Excess reaction of SiYₙZₚ (n=2) yields the following polymeric chain.

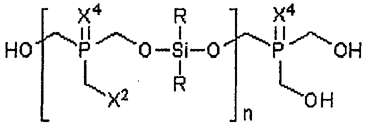

Excess reaction of SiYₙZₚ (n=3) yields the following polymeric chain.

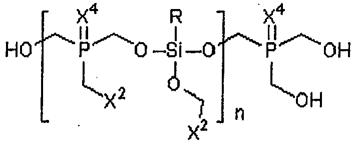

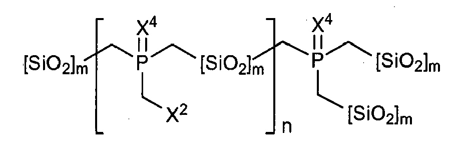

In the compounds obtained with the above-indicated method, =X⁴ has the expected properties of transition metal atom coordination, that is to say a Lewis acid/Lewis base type interaction (X⁴ - metal centre).

A soluble compound according to the invention may be easily determined by NMR (nuclear magnetic resonance) and mass spectroscopy. The number of monomer units may thus be clearly defined and it allows demonstrating that it is typically possible to obtain oligomers comprising from 8 to 10 "monomeric" units.

### FIGURES

**Figure 1** diagrammatically represents a purification process comprising a complex according to the invention.

**Figure 2** diagrammatically represents a chemical synthesis method using, as catalytic agent, a complex according to the invention;

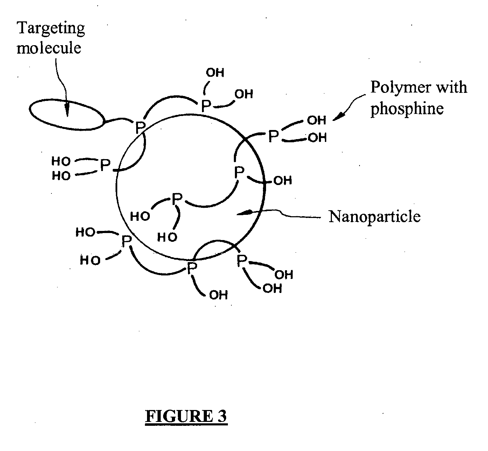

**Figure 3** diagrammatically represents a complex comprising metal atoms in the form of nanoparticles and capable of creating bonds with a target entity.

The invention will be better understood and other advantages will become apparent on reading the description which will follow, given without implied limitation.

### EXAMPLES

### Synthesis of a compound according to the invention

In general, compounds according to the invention may be synthesized according to at least one of the following methods.

According to a first method, a solution is made with 25g of tris(hydroxymethyl)phosphine (marketed THPC) in 250mL of water, cooled to 0°C. An aqueous solution of NaOH is then added under stirring (5.7g in 100mL of water). The reaction is completed within 5 minutes after the end of the addition of the base, as shown by the total disappearance of the feedstock's signal (+25 ppm in NMR ³¹P) and the appearance of the end product's signal THP at -25 ppm.

The reaction mixture then contains an equimolar proportion of THP and CH₂O (formaldehyde).

In a second step, carried out in the same vessel, the mixture is allowed to react with the desired amine derivative.

According to a second method, a solution is made with marketed THPC, water and a suitable amount of amine derivative.

### Example 1

### Compound comprising a polymer P1 wherein X1 and X2 represent -NH₂, X3 represents -NH or -NR³- with R³ being of formula (I), and =X4 represents an electron pair

A polymer synthesis is carried out using marketed THPC (80% mass in H₂O) treated as above-indicated.

Solid ammonium salt NH₄Cl (6.1g) is added to the mixture.

The obtained polymer P1 precipitates very quickly in a quantitative way. It is insoluble in usual organic solvents.

### Example 2

### Compound comprising a polymer P2 wherein X1 and X2 represent -NH₂, X3 represents -NH or -NR³- with R³ being of formula (I), and =X4 represents =O

At the end of the preceding synthesis, the obtained suspension is allowed to react with H₂O₂ (6mL in 40 mL of water) during 1 hour. The resulting solid, i.e. the obtained polymer P2, is rinsed with water and dried.

### Example 3

### Compound comprising a polymer P3 wherein X1 and X2 represent -NHC₈H₁₇, X3 represents -NC₈H₁₇-, and =X4 represents an electron pair

According to the first method, the mixture obtained at the end of the first step from 2.39g of THPC (10mmol) is allowed to stoichiometrically react (1.5 equivalent compared with THP) with 2.48mL of octylamine (C₈H₁₇NH₂). A precipitate forms. 10 minutes after, this precipitate is centrifuged, rinsed with water, then with ethanol, and dried. This compound is soluble in organic solvents and is characterized by a signal at -60ppm in NMR ³¹P.

### Example 4

### Compound comprising a polymer P4 wherein X1 and X2 represent - NHC₁₂H₂₅-, X3 represents -NC₁₂H₂₅, and =X4 represents an electron pair

According to the first method, the mixture (Vₜₒₜₐₗ H₂O = 50mL) obtained at the end of the first step from 2.39g of THPC (10mmol) is allowed to stoichiometrically react (1.5 equivalent compared with THP) with 2.77g of dodecylamine (C₁₂H₂₅HN₂) diluted in 50mL of toluene. This reaction is vigorously stirred overnight. The polymer is obtained after settling of both phases. The organic phase is vacuum dried, leaving an oily polymer characterized by a signal at -65ppm in NMR ³¹P.

### Example 5

### Compound obtained according to a sol-gel reaction.

The compound is obtained as described in Example 1, but a silica precursor is used as second reactant.

### Example 6

### Synthesis of a silica supported compound.

A silica supported polymer is made according to the same process as Example 1 but in the presence of silica that was previously heated at 500°C overnight. The particle size is dependent of the silica gel used. Approximately, 10-20% of silica is used with respect to the THPC used.

## Claims

1. Use of a compound comprising at least one polymeric chain, said polymeric chain incorporating phosphorus atoms and consisting, in all or in part, of identical or different repeated units, each of said units being represented by the following formula (I): 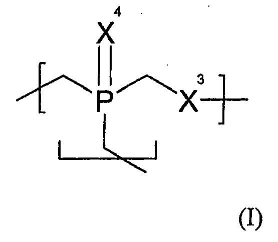 wherein:
- X³ represents:
-O-[Si(R¹R²)O]- with R¹ and R² being, independently of each other, a C₁-C₃₀ alkyl or alkoxy group, a C₅-C₃₀ aryl group, or
- a mono- or polyorganosilicate derived radical or
-N(R³)- with R³ being -H, a C₁-C₃₀ alkyl group or a C₅-C₃₀ aryl group, optionally substituted with -OH or -NH₂, or at least one unit of general formula (I), and
- =X⁴ represents an electron pair, =O, =S, =NR⁴ with R⁴ representing a C₁-C₃₀ alkyl group or a C₅-C₃₀ aryl group,
for complexing metal atoms.

2. The use according to the preceding claim, wherein said polymeric chain is liable to be obtained according to a method comprising at least the step of contacting a first reactant P(CH₂Z)₃, wherein each Z represents-OH , with a second reactant comprising at least two reactive sites chosen from "NH" and "SiOH", wherein each reactive site is liable to react with one first reactant,
wherein said first reactant and said reactive sites are used in an amount equal or greater than 1 equivalent, and more particularly ranging from 1 to 1.4 equivalents, and more preferably ranging from 1.1 to 1.3 equivalents.

3. The use according to the preceding claim, wherein said second reactant is chosen from NH₂R with R representing a C₁-C₃₀ alkyl, optionally substituted with -OH or NH₂, or a C₅-C₃₀ aryl group; silica derivatives such as SiYₙZ_{P}, with n+p=4, and Y figuring a radical chosen from OH and Z figuring a radical chosen from a C₁-C₅ alkyl group or a C₅-C₃₀ aryl group.

4. The use according to anyone of claims 1 to 3, wherein said polymeric chain is represented by the following formula (II): 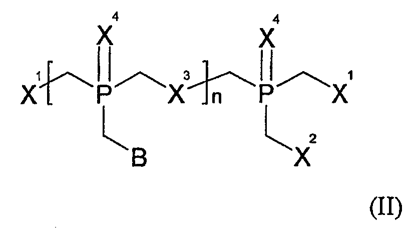 wherein:
- n ranges from 1 to 10⁶, in particular from 10 to 10⁵, and more particularly from 100 to 10⁴,
- X³ and =X⁴ are as defined in claim 1, with each X³ being identical to each other when n is greater than 1, and when X³ represents -NR³- with R³ being at least one unit of general formula (I) then the free extremities of said at least one unit which are not engaged in said polymeric chain are substituted with X¹ or X² as defined below,
- X¹ and X² represent, independently of each other, -OH, NH₂, -NHR⁵, or -OSiR⁶R⁷(OH), with R⁵, R⁶ and R⁷ being, independently of each other, a C₁-C₃₀ alkyl group or a C₅-C₃₀ aryl group, or R⁶ being as previously defined and R⁷ figuring -OCH₂-B, with B representing a radical of formula (III): 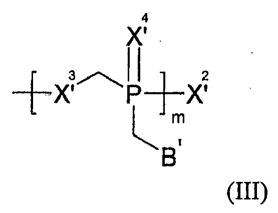 wherein:
- m ranges from 0 to 10⁶, in particular from 10 to 10⁵, and more particularly from 100 to 10⁴, and
- B' represents B, with each B being identical to each other when m is different from 0,
- X'² represents X² as above-defined, X'³ represents X³ as above-defined and =X'⁴ represents =X⁴ as defined in claim 1, with each X'³ being identical to each other when m is different from 0.

5. The use according to the preceding claim, wherein X¹ and X² represent, independently of each other, -OH, -NHR⁵, or -OSiR⁶R⁷(OH), with R⁵, R⁶ and R⁷ being as defined in claim 4.

6. The use according to anyone of the preceding claims, wherein said polymeric chain is represented by the following formula (IV): 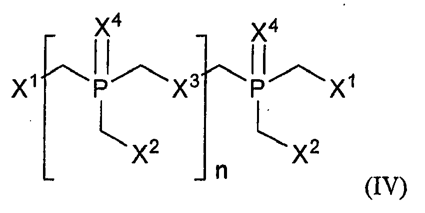 wherein:
- n, X¹, X², X³ and =X⁴ are as defined in claims 1, 4 or 5.

7. The use according to anyone of claims 1 to 5, wherein said polymeric chain is represented by the following formula (V): 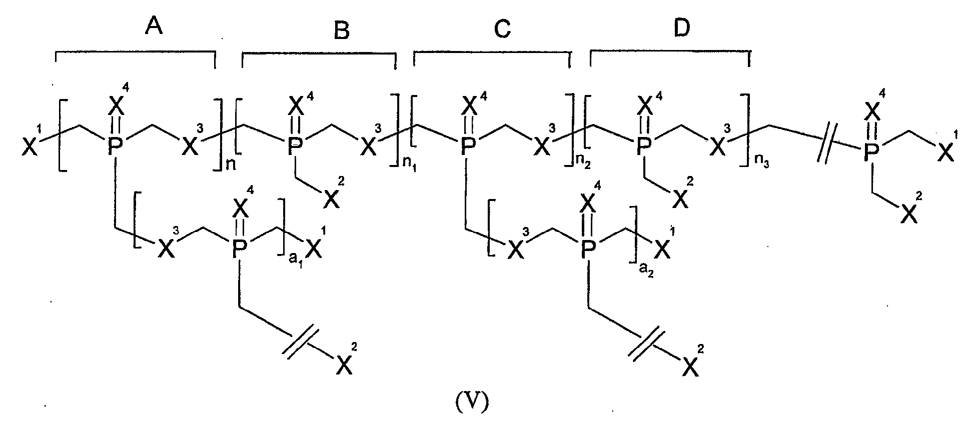 wherein:
- n, X¹, X², X³ and =X⁴ are as defined in claims 1, 4 or 5,
- n₁, n₂ and n₃ range, independently of each other, from 0 to 10⁶, in particular from 10 to 10⁵, and more particularly from 100 to 10⁴, and
- a₁ and a₂ range from 1 to 10⁶, in particular from 10 to 10⁵, and more particularly from 100 to 10⁴,
- -//- figures an optional continuation of said polymeric chain, and
- A, B, C and D are randomly or sequentially distributed.

8. The use according to anyone of claims 4 to 7, wherein X¹ and X² are identical.

9. The use according to anyone of the preceding claims, wherein =X⁴ represents =O, =S or an electron pair, and more particularly is an electron pair.

10. The use of anyone of previous claims wherein said compound is insoluble in water.

11. The use according to anyone of the preceding claims, wherein said polymeric chain is grafted onto a support of mineral or organic material.

12. The use according to the preceding claim, wherein said support of mineral material is chosen from the group consisting of a metal oxide, and in particular is TiO₂ or SiO₂.

13. The use according to claim 11, wherein said support of organic material is chosen from the group consisting of cellulose fibers, paper, plastic, mineral materials functionalized with APS or the like, amine containing reagent, amine containing organic polymer, or biological amine containing polymers.

14. The use according to anyone of the preceding claims, for complexing metal atoms from water.

15. The use according to anyone of the preceding claims, for detecting metal atoms.

16. The use according to anyone of the preceding claims, wherein the metal atoms are chosen from an oxide or a salt or a neutral solvate of a paramagnetic metal of atomic number 21-29, 42- 44 or 58-70 or a radionuclide chosen from ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh, ¹⁸⁸Re, ⁶⁰Cu, ⁶²Cu, ⁶⁴Cu, ⁶⁷CU, ⁹⁰Y, ¹⁵⁹Gd, ¹⁴⁹Pr and ¹⁶⁶Ho, or an ion of a heavy metal of atomic number 21-31, 39-49, 50, 56-80, 82, 83 or 90.

17. A metal-based coordination complex comprising at least one metal atom complexed via coordination bonds with at least one compound as defined according to anyone of claims 1 to 13.

18. A stabilized nanoparticle of metal atoms coated at least in part with at least one compound as defined according to anyone of claims 1 to 13.

19. A compound for medical imaging comprising at least one metal-based coordination complex according to claim 17 or at least one stabilized nanoparticle of metal atoms according to claim 18.

20. A catalytic agent comprising at least one metal-based coordination complex according to claim 17 or at least one stabilized nanoparticle of metal atoms according to claim 18.

21. A water insoluble compound comprising at least one polymeric chain, said polymeric chain incorporating phosphorus atoms and being represented by the following formula (II): 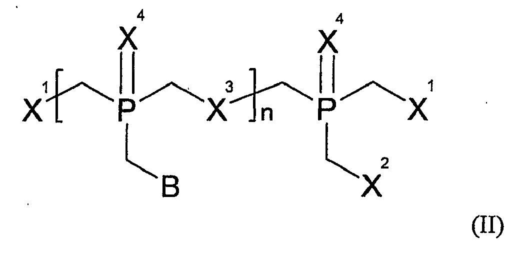 wherein:
- n, B, X¹, and X² are as defined in claim 4 or 5,
with each X³ being identical relative to each other when n is greater than 1, and X³ represents:
-O-[Si(R¹R²)O]- with R¹ and R² being, independently of each other, a C₁-C₃₀ alkyl or alkoxy group, a C₅-C₃₀ aryl group, or
- a mono- or polyorganosilicate derived radical or
-N(R³)- with R³ being a C₁ or C₃-C₅ alkyl group or a C₅-C₃₀ aryl group, optionally substituted with -OH or -NH₂, or at least one unit of general formula (I), 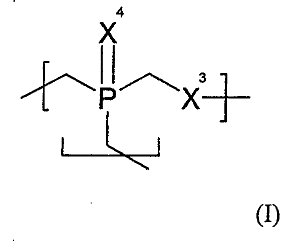
and
- =X⁴ is an electron pair, or =O.

22. The compound according to the preceding claim, wherein said polymeric chain is liable to be obtained according to a method comprising at least the step of contacting a first reactant P(CH₂Z)₃, wherein each Z represents -OH, with a second reactant comprising at least two reactive sites chosen from "NH" and "SiOH", wherein each reactive site is liable to react with one first reactant,
wherein said first reactant and said reactive sites are used in an amount equal or greater than 1 equivalent, and more particularly ranging from 1 to 1.4 equivalents, and more preferably ranging from 1.1 to 1.3 equivalents.

## Patentansprüche

1. Verwendung einer Verbindung, umfassend mindestens eine Polymerkette, wobei die Polymerkette Phosphoratome enthält und vollkommen oder teilweise aus identischen oder unterschiedlichen Wiederholungseinheiten besteht, wobei jede der Einheiten durch die folgende Formel (I) dargestellt wird: 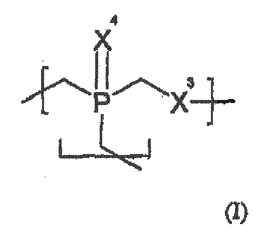 wobei:
- X³ darstellt:
- O-[Si(R¹R²)O]-, wobei R¹ und R² unabhängig voneinander ein C₁-C₃₀-Alkyl- oder
- Alkoxyrest, ein C₅-C₃₀-Arylrest sind, oder
- einen von Mono- oder Polyorganosilikat abgeleiteten Rest oder
- N(R³)-, wobei R³ -H, ein C₁-G₃₀-Alkylrest oder ein C₅-C₃₀-Arylrest ist, gegebenenfalls substituiert mit -OH oder -NH₂, oder mindestens eine Einheit der allgemeinen Formel (I), und
- =X⁴ ein Elektronenpaar, =O, =S, =NR⁴ darstellt, wobei R⁴ einen C₁-C₃₀-Alkylrest oder einen C₅-C₃₀-Arylrest darstellt,
zum Komplexieren von Metallatomen.

2. Verwendung gemäß dem vorhergehenden Anspruch, wobei die Polymerkette gemäß einem Verfahren erhalten werden kann, das mindestens den Schritt von Inkontaktbringen eines ersten Reaktanten P(CH₂Z)₃, wobei jedes Z -OH darstellt, mit einem zweiten Reaktanten, umfassend mindestens zwei reaktive Stellen, welche aus "NH" und "SiOH" ausgewählt sind, umfasst, wobei sich jede reaktive Stelle mit einem ersten Reaktanten umsetzen kann,
wobei der erste Reaktant und die reaktiven Stellen in einer Menge verwendet werden, die gleich oder größer als 1 Äquivalent ist und stärker besonders in einem Bereich von 1 bis 1,4 Äquivalenten liegt und stärker bevorzugt in einem Bereich von 1,1 bis 1,3 Äquivalenten liegt.

3. Verwendung gemäß dem vorhergehenden Anspruch, wobei der zweite Reaktant ausgewählt ist aus NH₂R, wobei R ein C₁-C₃₀-Alkyl, gegebenenfalls substituiert mit -OH oder -NH₂, oder einen C₅-C₃₀-Arylrest darstellt; Siliciumdioxid-Derivaten wie SiYₙZₚ, wobei n+p=4 gilt und Y einen Rest darstellt, der aus OH ausgewählt ist, und Z einen Rest darstellt, der aus einem C₁-C₅-Alkylrest oder einem C₅-C₃₀-Arylrest ausgewählt ist.

4. Verwendung gemäß einem der Ansprüche 1 bis 3, wobei die Polymerkette durch die folgende Formel (II) darstellt wird: 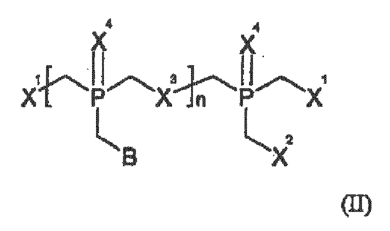 wobei:
- n in einem Bereich von 1 bis 10⁶, insbesondere 10 bis 10⁵ und stärker besonders 100 bis 10⁴ liegt,
- X³ und =X⁴ wie in Anspruch 1 definiert sind, wobei jedes X³ identisch mit jedem anderem X³ ist, wenn n größer als 1 ist, und wenn X³ -NR³- darstellt, wobei R³ mindestens eine Einheit der allgemeinen Formel (I) ist, dann die freien Reste der mindestens einen Einheit, die nicht an der Polymerkette beteiligt sind, mit X¹ oder X² wie nachstehend definiert substituiert sind,
- X¹ und X² unabhängig voneinander -OH, NH₂, -NHR⁵ oder -OSiR⁶R⁷(OH) darstellen, wobei R⁵, R⁶ und R⁷ unabhängig voneinander ein C₁-C₃₀-Alkylrest oder ein C₅-C₃-Arylrest sind oder R⁶ wie vorstehend definiert ist und R⁷ -OCH₂-B darstellt, wobei B einen Rest der Formel (III) darstellt: 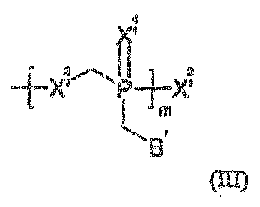 wobei:
- m in einem Bereich von 0 bis 10⁶, insbesondere 10 bis 10⁵ und stärker besonders 100 bis 10⁴ liegt, und
- B' B darstellt, wobei jedes B identisch mit jedem anderen B ist, wenn m verschieden von 0 ist,
- X'² X² wie vorstehend definiert darstellt, X'³ X³ wie vorstehend definiert darstellt und =X'⁴ =X⁴ wie in Anspruch 1 definiert darstellt, wobei jedes X'³ identisch mit jedem anderem X'³ ist, wenn m verschieden von 0 ist.

5. Verwendung gemäß dem vorhergehenden Anspruch, wobei X¹ und X² unabhängig voneinander -OH, -NHR⁵ oder -OSiR⁶R⁷(OH) darstellen, wobei R⁵, R⁶ und R⁷ wie in Anspruch 4 definiert sind.

6. Verwendung gemäß einem der vorhergehenden Ansprüche, wobei die Polymerkette durch die folgende Formel (IV) dargestellt wird: 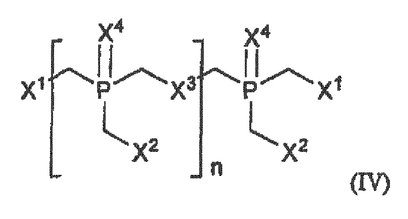 wobei:
- n, X¹, X², X³ und =X⁴ wie in den Ansprüchen 1, 4 oder 5 definiert sind.

7. Verwendung gemäß einem der Ansprüche 1 bis 5, wobei die Polymerkette durch die folgende Formel (V) dargestellt wird: 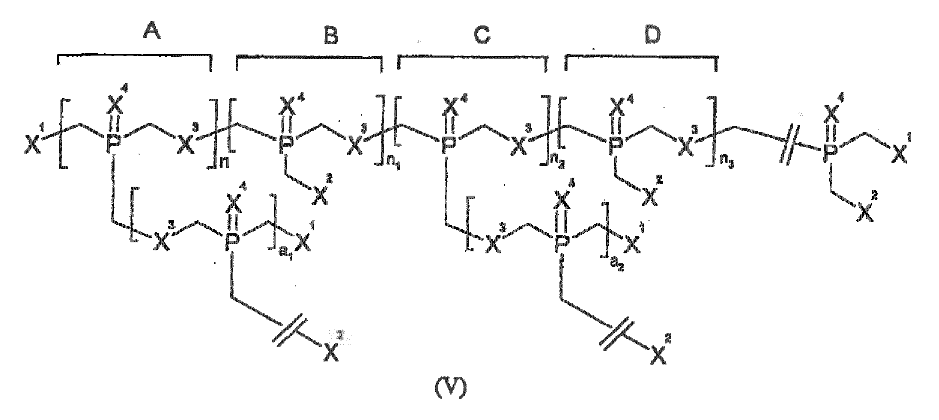 wobei:
- n, X¹, X², X³ und =X⁴ wie in den Ansprüchen 1,4 oder 5 definiert sind,
- n₁, n₂ und n₃ unabhängig voneinander in einem Bereich von 0 bis 10⁶, insbesondere 10 bis 10⁵ und stärker besonders 100 bis 10⁴ liegen, und
- a₁ und a₂ in einem Bereich von 1 bis 10⁶, insbesondere 10 bis 10⁵ und stärker besonders 100 bis 10⁴ liegen,
- -//- eine optionale Fortsetzung der Polymerkette darstellt, und
- A, B, C und D statistisch oder sequenziell verteilt sind.

8. Verwendung gemäß einem der Ansprüche 4 bis 7, wobei X¹ und X² identisch sind.

9. Verwendung gemäß einem der vorhergehenden Ansprüche, wobei =X⁴ =O, =S oder ein Elektronenpaar darstellt und stärker besonders ein Elektronenpaar ist.

10. Verwendung nach einem der vorstehenden Ansprüche, wobei die Verbindung unlöslich in Wasser ist.

11. Verwendung gemäß einem der vorhergehenden Ansprüche, wobei die Polymerkette auf einen Träger aus Mineral- oder organischem Material gepfropft ist.

12. Verwendung gemäß dem vorhergehenden Anspruch, wobei der Träger aus Mineralmaterial aus der Gruppe ausgewählt ist, die aus einem Metalloxid besteht, und insbesondere TiO₂ oder SiO₂ ist.

13. Verwendung gemäß Anspruch 11, wobei der Träger aus organischem Material aus der Gruppe ausgewählt ist, die aus Cellulosefasern, Papier, Kunststoff, Mineralmaterialien, die funktionalisiert sind mit ASP oder dergleichen, aminhaltigem Reagenz, aminhaltigem organischem Polymer oder biologischen aminhaltigen Polymeren besteht.

14. Verwendung gemäß einem der vorhergehenden Ansprüche zum Komplexieren von Metallatomen aus Wasser.

15. Verwendung gemäß einem der vorhergehenden Ansprüche zum Nachweisen von Metallatomen.

16. Verwendung gemäß einem der vorhergehenden Ansprüche, wobei die Metallatome aus einem Oxid oder einem Salz oder einem neutralen Solvat eines paramagnetischen Metalls mit der Ordnungszahl 21-29, 42-44 oder 58-70 oder einem Radionuklid, ausgewählt aus ⁹⁹Tc, ¹¹⁷Sn ¹¹¹In, ⁹⁷Ru, ⁶⁷Ga, ⁶⁸Ga, ⁸⁹Zr ¹⁷⁷Lu ⁴⁷Sc, ¹⁰⁵Rh, ¹⁸⁸Re, ⁶⁰Cu, ⁶²Cu, ⁶⁴Cu, ⁶⁷Cu, ⁹⁰Y, ¹⁵⁹Gd, ¹⁴⁹Pr und ¹⁶⁶Ho, oder einem Ion eines Schwermetalls mit der Ordnungszahl 21-31, 39-49, 50, 56-80, 82, 83 oder 90 ausgewählt sind.

17. Koordinationskomplex auf Metall-Basis, umfassend mindestens ein Metallatom, das über Koordinationsbindungen mit mindestens einer Verbindung wie gemäß einem der Ansprüche 1 bis 13 definiert komplexiert ist.

18. Stabilisiertes Nanoteilchen aus Metallatomen, das mindestens teilweise mit mindestens einer Verbindung wie gemäß einem der Ansprüche 1 bis 13 definiert überzogen ist.

19. Verbindung für medizinische Bildgebung, umfassend mindestens einen Koordinationskomplex auf Metall-Basis gemäß Anspruch 17 oder mindestens ein stabilisiertes Nanoteilchen aus Metallatomen gemäß Anspruch 18.

20. Katalytisches Mittel, umfassend mindestens einen Koordinationskomplex auf Metall-Basis gemäß Anspruch 17 oder mindestens ein stabilisiertes Nanoteilchen aus Metallatomen gemäß Anspruch 18.

21. Wasserunlösliche Verbindung, umfassend mindestens eine Polymerkette, wobei die Polymerkette Phosphoratome enthält und durch die folgende Formel (II) dargestellt wird: 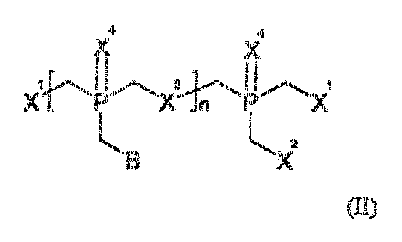 wobei:
- n, B, X¹ und X² wie in Anspruch 4 oder 5 definiert sind,
wobei jedes X³ relativ zueinarider identisch ist, wenn n größer als 1 ist, und X³ darstellt:
-O-[Si(R¹R²)O]-, wobei R¹ und R² unabhängig voneinander ein C₁-C₃₀-Alkyl- oder -Alkoxyrest, ein C₅-C₃₀-Arylrest sind, oder
- einen von Mono- oder Polyorganosilikat abgeleiteten Rest oder
-N(R³)-, wobei R³ ein C₁- oder C₃-C₅-Alkylrest oder ein C₅-C₃₀-Arylrest ist, gegebenenfalls substituiert mit -OH oder -NH₂, oder mindestens eine Einheit der allgemeinen Formel (I), 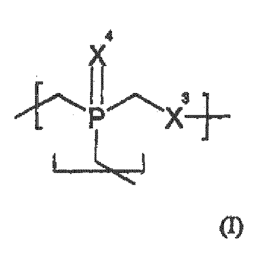
und
- =X⁴ ein Elektronenpaar oder =O ist.

22. Verbindung gemäß dem vorhergehenden Anspruch, wobei die Polymerkette gemäß einem Verfahren erhalten werden kann, das mindestens den Schritt von Inkontaktbringen eines ersten Reaktanten P(CH₂Z)₃, wobei jedes Z -OH darstellt, mit einem zweiten Reaktanten, umfassend mindestens zwei reaktive Stellen, welche aus "NH" und "SiOH" ausgewählt sind, umfasst, wobei sich jede reaktive Stelle mit einem ersten Reaktanten umsetzen kann,
wobei der erste Reaktant und die reaktiven Stellen in einer Menge verwendet werden, die gleich oder größer als 1 Äquivalent ist und stärker besonders in einem Bereich von 1 bis 1,4 Äquivalenten liegt und stärker bevorzugt in einem Bereich von 1,1 bis 1,3 Äquivalenten liegt.

## Revendications

1. Utilisation d'un composé comprenant au moins une chaîne polymère, ladite chaîne polymère incorporant des atomes de phosphore et étant constituée, en totalité ou en partie, de motifs répétitifs identiques ou différents, chacun desdits motifs étant représenté par la formule (I) suivants : 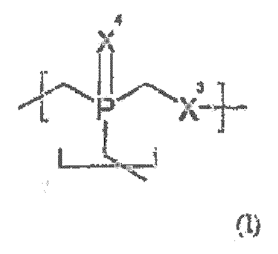 dans laquelle :
- X³ représente :
- O-[Si(R¹R²)O] - avec R¹ et R² étant, indépendamment l'un de l'autre, un groupe alkyle ou alcoxy en C₁ à C₃₀, un groupe aryle en C₅ à C₃₀, ou
- un radical dérivé d'un mono- ou polyorganosilicate ou
- N (^{R}3) - avec R³ étant -H, un groupe alkyle en C₁ à C₃₀ ou un groupe aryle en C₅ à C₃₀, optionnellement substitué par -OH ou -NH₂, ou au moins un motif de formule générale (I), et
- =X⁴ représente une paire d'électrons, =O, =S, =NR⁴ avec R⁴ représentant un groupe alkyle en C₁ à C₃₀ ou un groupe aryle en C₅ à C₃₀,
pour la complexation d'atomes de métal.

2. Utilisation selon la revendication précédente, dans laquelle ladite chaîne polymère est susceptible d'être obtenue selon un procédé comprenant au moins l'étape de mise en contact d'un premier réactif P(CH₂Z)₃, formule dans laquelle chaque Z représente -OH, avec un deuxième réactif comprenant au moins deux sites réactifs choisis parmi « NH » et « SiOH », dans lequel chaque site réactif est susceptible de réagir avec un premier réactif,
dans laquelle ledit premier réactif et lesdits sites réactifs sont utilisés en une quantité supérieure ou égale à 1 équivalent, et plus particulièrement située dans la plage de 1 à 1,4 équivalent, et plus préférablement située dans la plage de 1,1 à 1,3 équivalent.

3. Utilisation selon la revendication précédente, dans laquelle ledit deuxième réactif est choisi parmi NH₂R avec R représentant un groupe alkyle en C₁ à C₃₀, optionnellement substitué par -OH ou -NH₂, ou un groupe aryle en C₅ à C₃₀ ; des dérivés de la silice tels que SiYₙZₚ, avec n + p = 4, et Y désignant un radical choisi parmi OH et Z désignant un radical choisi parmi un groupe alkyle en C₁ à C₅ ou un groupe aryle en C₅ à C₃₀.

4. Utilisation selon l'une quelconque des revendications 1 à 3, dans laquelle ladite chaîne polymère est représentée par la formule (II) suivants : 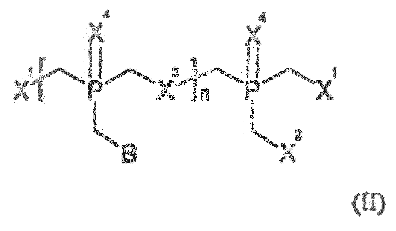 dans laquelle :
n est situé dans la plage de 1 à 10⁶, en particulier de 10 à 10⁵, et plus particulièrement de 100 à 10⁴,
- X³ et =X⁴ sont tels que définis dans la revendication 1, avec tous les X³ étant identiques les uns aux autres lorsque n est supérieur à 1, et lorsque X³ représente -NR³- avec R³ étant au moins un motif de formule générale (1) alors les extrémités libres dudit au moins un motif qui ne sont pas engagées dans ladite chaîne polymère sont substituées par X¹ ou X² tels que définis ci-dessous,
- X¹ et X² représentent, indépendamment l'un de l'autre, -OH, NH₂, -NHR⁵ ou -OSiR⁶R⁷(OH), avec R⁵, R⁶ et R⁷ étant, indépendamment l'un de l'autre, un groupe alkyle en C₁ à C₃₀ ou un groupe aryle en C₅ à C₃₀, ou R⁶ étant tel que défini précédemment et R⁷ désignant -OCH₂-B, avec B représentant un radical de formule (III) : 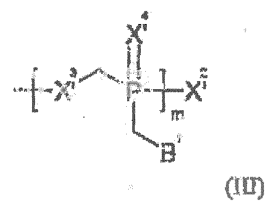 dans laquelle :
- m est situé dans la plage de 0 à 10⁶, en particulier de 10 à 10⁵, et plus particulièrement de 100 à 10⁴ et
- B' représente B, avec tous les B étant identiques les uns aux autres lorsque m est différent de 0
- X'² représente X² tel que défini ci-dessus, X'³ représente X³ tel que défini ci-dessus et =X'⁴ représente =X⁴ tel que défini dans la revendication 1, avec tous les X'³ étant identiques les uns aux autres lorsque m est différent de 0.

5. Utilisation selon la revendication précédente, dans laquelle X¹ et X² représentent, indépendamment l'un de l'autre, -OH, -NHR⁵ ou -OSiR⁶R⁷(OH), avec R⁵, R⁶ et R⁷ étant selon la revendication 4.

6. Utilisation selon l'une quelconque des revendications précédentes, dans laquelle ladite chaîne polymère est représentée par la formule (IV) suivants : 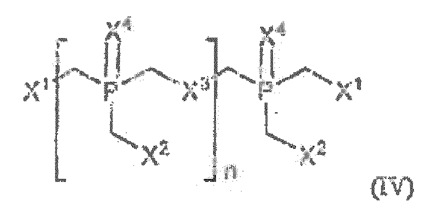 dans laquelle :
- n, X¹, X², X³ et =X⁴ sont selon les revendications 1, 4 ou 5.

7. Utilisation selon l'une quelconque des revendications 1 à 5, dans laquelle ladite chaîne polymère est représentée par la formule (V) suivante : 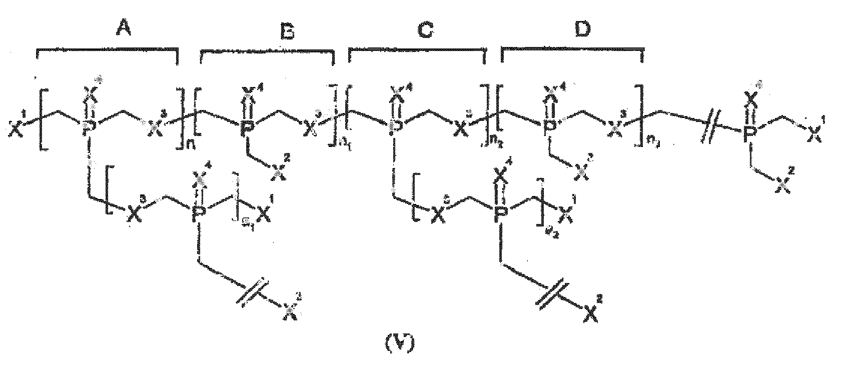 dans laquelle :
- n, X¹, X², X³ et =X⁴ sont selon les revendications 1, 4 ou 5,
- n₁, n₂ et n₃ sont situés dans la plage, indépendamment les uns des autres, de 0 à 10⁶, en particulier de 10 à 10⁵, et plus particulièrement de 100 à 10⁴, et
- a₁ et a₂ sont situés dans la plage de 1 à 10⁶, en particulier de 10 à 10⁵, et plus particulièrement de 100 à 10⁴,
- -//- désigne le prolongement optionnel de ladite chaîne polymère, et
- A, B, C et D sont distribués de manière aléatoire ou séquentielle.

8. Utilisation selon l'une quelconque des revendications 4 à 7, dans laquelle X¹ et X² sont identiques.

9. Utilisation selon l'une quelconque des revendications précédentes, dans laquelle =X⁴ représente =O, =S ou une paire d'électrons, et représente plus particulièrement une paire d'électrons.

10. Utilisation selon l'une quelconque des revendications précédentes, dans laquelle ledit composé est insoluble dans l'eau.

11. Utilisation selon l'une quelconque des revendications précédentes, dans laquelle ladite chaîne polymère est greffée sur un support constitué d'un matériau minéral ou organique.

12. Utilisation selon la revendication précédente, dans laquelle ledit support constitué d'un matériau minéral est choisi dans le groupe constitué d'un oxyde de métal, et est en particulier du TiO₂ ou du SiO₂.

13. Utilisation selon la revendication 11, dans laquelle ledit support constitué d'un matériau organique est choisi dans le groupe constitué de fibres de cellulose, de papier, de plastique, de matériaux minéraux fonctionnalisés avec de l'APS ou autres, d'un réactif aminé, d'un polymère organique aminé ou de polymères aminés biologiques.

14. Utilisation selon l'une quelconque des revendications précédentes, pour la complexation d'atomes de métal dans l'eau.

15. Utilisation selon l'une quelconque des revendications précédentes, pour détecter des atomes de métal.

16. Utilisation selon l'une quelconque des revendications précédentes, dans laquelle les atomes de métal sont choisis parmi un oxyde ou un sel ou un solvate neutre d'un métal paramagnétique de numéro atomique 21-29, 42-44 ou 58-70 ou un radionucléide choisi parmi ⁹⁹Tc, ¹¹⁷Sn, ¹¹¹In, ⁹⁷Ru, ⁶⁷Ga, ^{6B}Ga, ⁸⁹Zr, ¹⁷⁷Lu, ⁴⁷Sc, ¹⁰⁵Rh, ¹⁸⁸Re, ⁶⁰Cu, ⁶²Cu, ⁶⁴Cu, ⁶⁷Cu, ⁹⁰Y, ¹⁵⁹Gd, ¹⁴⁹Pr et ¹⁶⁶Ho, ou un ion de métal lourd de numéro atomique 21-31, 39-49, 50, 56-80, 82, 83 ou 90.

17. Complexe de coordination à base de métal comprenant au moins un atome de métal complexé via des liaisons de coordination à au moins un composé tel que défini selon l'une quelconque des revendications 1 à 13.

18. Nanoparticule stabilisée d'atomes de métal revêtue au moins en partie avec au moins un composé tel que défini selon l'une quelconque des revendications 1 à 13.

19. Composé destiné à l'imagerie médicale comprenant au moins un complexe de coordination à base de métal selon la revendication 17 ou au moins une nanoparticule stabilisée d'atomes de métal selon la revendication 18.

20. Agent catalytique comprenant au moins un complexe de coordination à base de métal selon la revendication 17 ou au moins une nanoparticule stabilisée d'atomes de métal selon la revendication 18.

21. Composé insoluble dans l'eau comprenant au moins une chaîne polymère, ladite chaîne polymère incorporant des atomes de phosphore et étant représentée par la formule (II) suivante : 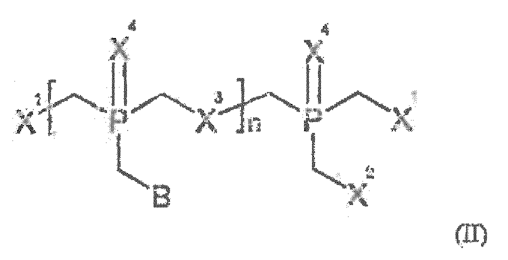 dans laquelle :
- n, B, X¹ et X² sont tels que définis dans la revendication 4 ou 5,
avec tous les X³ étant identiques les uns aux autres lorsque n est supérieur à 1, et X³ représente :
- O-[Si(R¹R²)O]- avec R¹ et R² étant, indépendamment l'un de l'autre, un groupe alkyle ou alcoxy en C₁ à C₃₀, un groupe aryle en C₅ à C₃₀, ou
- un radical dérivé d'un mono- ou polyorganosilicate ou
- N(R³)- avec R³ étant un groupe alkyle en C₁ ou en C₃ à C₅ ou un groupe aryle en C₅ à C₃₀, optionnellement substitué par -OH ou -NH₂, ou au moins un motif de formule générale (I), 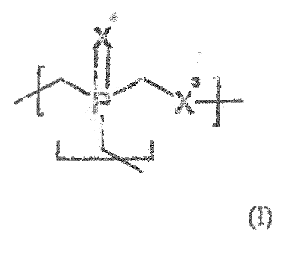
et
- =X⁴ représente une paire d'électrons, ou =O,

22. Composé selon la revendication précédente, dans lequel ladite chaîne polymère est susceptible d'être obtenue selon un procédé comprenant au moins l'étape de mise en contact d'un premier réactif P(CH₂Z)₃, formule dans laquelle chaque Z représente -OH, avec un deuxième réactif comprenant au moins deux sites réactifs choisis parmi « NH » et « SiOH », dans lequel chaque site réactif est susceptible de réagir avec un premier réactif,
dans lequel ledit premier réactif et lesdits sites réactifs sont utilisés en une quantité supérieure ou égale à 1 équivalent, et plus particulièrement située dans la plage de 1 à 1,4 équivalent, et plus préférablement située dans la plage de 1,1 à 1,3 équivalent.
